# EUROPEAN PATENT APPLICATION

(11) **EP 4 437 935 A1**
(43) Date of publication of application: **02.10.2024**
(21) Application number: 23305467.5
(22) Date of filing: 31.03.2023
(51) Int. Cl.: A61B 3/00, A61B 3/028, A61B 3/032, A61B 3/04, A61B 3/06

(54) **OPTOMETRY DEVICE FOR TESTING THE EYES OF AN INDIVIDUAL, ASSOCIATED METHOD AND COMPUTER PROGRAM PRODUCT**

(71) Applicant: Essilor International, 94220 Charenton-Le-Pont (FR)
(72) Inventor: STARYNKEVITCH, Hélène, 78000 Versailles (FR)
(74) Representative: Jacobacci Coralis Harle

(57) **Abstract**

The invention relates to an optometry device for testing the eyes of an individual, comprising a computer with one or more processors programmed to perform a sequence of subjective tests comprising at least a first and a second predetermined subjective test, leading to the determination of values of one or more refractive features of one or more of the eyes of the individual wherein said one or more processors are programmed to:
a) perform said first predetermined subjective test, thereby determining a provisory value of a first refraction feature of the eye of the individual,
b) at the end of said first predetermined subjective test, perform a verification test for detecting a potential error in said provisory value of the first refraction feature determined in step a),
c) based on the result of said verification test, determine a next subjective test to be performed or ending the sequence of subjective tests.

## Description

### TECHNICAL FIELD OF THE INVENTION

The invention relates to an optometry device for testing the eyes of an individual, an associated method and a computer program product.

### BACKGROUND INFORMATION AND PRIOR ART

Manufacturing an ophthalmic lens adapted to improve the vision of an individual requires the determination of a value of an optical feature of said ophthalmic lens adapted to the individual. To determine such a value of said optical feature, with the purpose of manufacturing an ophthalmic lens, the eye-care professional usually performs a sequence of subjective tests on the individual thanks to an appropriate optometry device to determine one or more refraction feature of the eye of the individual. The optical feature of the ophthalmic lens is determined based on this refraction feature of the eye. Numerous documents describe devices and methods for determining this value of the optical feature of the ophthalmic lens. This optical feature may include for example sphere power, cylinder power or cylinder axis.

Typically, each subjective test comprises several steps, hereafter called trials, during each of which the individual is required to compare two different optical situations taking into account a trial value of an optical feature of a trail lens placed in front of the eye of the individual. Depending on the feedback of the individual on this comparison, designated in the following as the answer of the individual, the eye-care professional increments the trial value and, in the next trial of the subjective test, presents the individual with two new different optical situations based on the incremented trial value. This process is repeated until a specific answer or combination of answers is given by the individual. The refraction feature of the eye is then usually determined as a function of the test value used in the last trial of said subjective test.

During a standard sequence of subjective tests, the sequence is predetermined, meaning that it comprises at least a first and a second predetermined subjective test that are planned to be performed successively.

The sequence of subjective tests is carried out, even if the result of one of the predetermined subjective tests is not consistent with data relative to the individual or with results of previously performed subjective test of the sequence. This may lead to errors.

To avoid such errors, the constant attention of the eye-care professional is needed to verify that the subjective tests converge towards a consistent value of the optical feature of the ophthalmic lens.

Moreover, additional tests have to be carried out, leading to time consuming refraction processes requiring a long time, even for individuals having a satisfactory consistence of the sequence of subjective tests. The long refraction time can be a cause of fatigue and weariness for both the individual and the eye-car professional and eventually induce errors.

### SUMMARY OF THE INVENTION

One object of the invention is to provide an optometry device ensuring that reliable and accurate values of one or more refraction features of one or more of the eyes of the individual are determined without requiring a constant attention from the eye-care professional, and in a fast and efficient way. The optical feature of the ophthalmic lens may then be determined accurately based of the refraction feature of the eye, and the ophthalmic lens may be manufacture accordingly.

To this end, the invention comprises an optometry device for testing the eyes of an individual, comprising a computer with one or more processors programmed to perform a sequence of subjective tests comprising at least a first and a second predetermined subjective test, leading to the determination of values of one or more refractive features of one or more of the eyes of the individual by:
a) performing said first predetermined subjective test, thereby determining a provisory value of a first refraction feature of the eye of the individual,
b) at the end of said first predetermined subjective test, performing a verification test for detecting a potential error in said provisory value of the first refraction feature determined in step a),
c) based on the result of said verification test, determining a next subjective test to be performed or ending the sequence of subjective tests.

Thanks to the optometry device of the invention, the sequence of subjective tests is adapted automatically depending on the results of the first subjective test. Potential errors may be detected and the next subjective test may be determined taking into account the risk of an error having occurred in the first subjective test. The effect of a potential error on the final refraction values of the eyes determined may be limited or eliminated. The consistence of the results obtained for a given first subjective test is checked in the verification step and the subsequent subjective test of the sequence takes into account the result of this verification step.

Said value of one or more refraction features of one or more of the eyes of the individual is determined taking into account said provisory value of the first refraction feature. The value of one or more refractive features of one or more of the eyes of the individual may be equal to the provisory value of the first refractive feature or may be determined through the sequence of subjective tests adapted taking into account the provisory value.

In particular, the subjective test considered comprises initially at least a first and a second predetermined subjective test. In the state of the art, the predetermined first and second predetermined subjective test would be performed successively, without taking into account the provisory value of the first refractive feature determined in the first predetermined subjective test to check for any inconsistencies.

In the optometry device of the invention, the verification test ensures that the provisory value is taken into account to modify the sequence if the result of the verification test suggests that this provisory value is not accurate.

According to further non limiting features of the device of the invention:
- in step b), said one or more processors are programmed to:
   * compare the provisory value of the first refraction feature determined through said first subjective test to a refraction threshold value,
      and/or
   * compare an acuity value of at least one of the eyes of the individual to an acuity threshold value;
- in step b), said one or more processors are programmed to detect a potential error in said provisory value:
   * when the first refraction feature is above said refraction threshold value or
   * when the acuity value is less than said acuity threshold value or
   * when the first refraction feature is above said refraction threshold value and the acuity value is less than said acuity threshold value;
- said one or more processors are programmed to determine said refraction threshold value based on a previous value of the first refraction feature determined before performing said first subjective test;
- said one or more processors are programmed to perform, in step b), an additional visual acuity test at the end of the first subjective test to determine said acuity value;
- when a potential error is detected in step b), said one or more processors are programmed to determine, in step c), the next subjective test to be performed by:
   * cancelling the first subjective test and performing either one of said second predetermined subjective test without any modification of the second predetermined subjective test and a modified second subjective test testing the same second refraction feature of the same eye with a different method,
      or
   * repeating said first subjective test without modification of the first subjective test or performing a modified first subjective test testing the same first refraction feature of the same eye with a different method;
- said first or second subjective test comprises a plurality of trials wherein a visual performance of the individual placed in two different optical situations is assessed and said optometry device is adapted to place said individual in different types of optical situations to perform said first or second subjective test, said predetermined first or second subjective test is associated with a predetermined type of optical situations and said modified first or second subjective test is associated with a different type of optical situations;
- said sequence of subjective tests comprises a third second predetermined subjective test, and, when a potential error is detected in step b), said one or more processors are programmed to determine, in step c), the next subjective test to be performed by performing said third predetermined subjective test without performing said second predetermined subjective test;
- said acuity threshold value comprises one of the following:
   * an initial value of visual acuity of at least one of the eyes of the individual determined before said first subjective test,
   * a reference value of visual acuity of the other eye of the individual determined before or after said first subjective test,
   * a target value of visual acuity;
- said one or more processors are programmed to perform the following step when no potential error is detected in step b), recording said provisory value as a confirmed value of said first refractive feature and performing said predetermined second subjective test;
- said one or more processors are programmed to perform an additional preliminary step before at least one of the subjective tests of said sequence of subjective tests, checking that predetermined conditions on the individual are met and determining whether said at least one subjective test is cancelled or modified based on the result of this additional preliminary step;
- in said additional preliminary step, said one or more processors are programmed to compare a value of the visual acuity of the right eye with a value of the visual acuity of the left eye or compare the age of the individual with a threshold value of age;

The invention also relates to a method for testing the eyes of an individual, implemented by a computer, comprising a sequence of subjective tests with at least a first and a second predetermined subjective test, leading to the determination of corresponding values of one or more refractive features of one or more of the eyes of the individual by:
a) performing said first subjective test, thereby determining a provisory value of a first refraction feature of the eye of the individual,
b) at the end of said first subjective test, performing a verification test for detecting a potential error in said provisory value of the first refraction feature determined in step a),
c) based on the result of said verification test, determining a next subjective test to be performed or ending the sequence of subjective tests.

In an embodiment of said method, said verification test comprises:
- comparing the provisory value of the first refraction feature determined through said first subjective test to a refraction threshold value,
   and/or
- comparing an final acuity value of at least one of the eyes of the individual to an acuity threshold value.

The invention also relates to a computer program product comprising instructions which, when the program is executed by a computer, cause the computer to carry out the steps of the method described above, leading to the determination of corresponding values of one or more refractive features of one or more of the eyes of the individual.

### DETAILED DESCRIPTION OF EXAMPLE(S)

The following description with reference to the accompanying drawings will make it clear what the invention consists of and how it can be achieved. The invention is not limited to the embodiment/s illustrated in the drawings. Accordingly, it should be understood that where features mentioned in the claims are followed by reference signs, such signs are included solely for the purpose of enhancing the intelligibility of the claims and are in no way limiting on the scope of the claims.

In the accompanying drawings:
- Figure 1 relates to a first general embodiment of the invention,
- Figures 2 and 3 relate to a second embodiment of the invention.

In the following description, identical or corresponding elements of each embodiment will be referred to with the same sign of reference and will not be described in detail each time.

The invention relates to an optometry device for testing the eyes of an individual.

According to the invention, the optometry device comprises a computer with one or more processors programmed to perform a sequence of subjective tests comprising at least a first and a second predetermined subjective test, leading to the determination of values of one or more refractive features of one or more of the eyes of the individual by:
a) performing said first predetermined subjective test, thereby determining a provisory value of a first refraction feature of the eye of the individual,
b) at the end of said first predetermined subjective test, performing a verification test for detecting a potential error in said provisory value of the first refraction feature determined in step a),
c) based on the result of said verification test, determining a next subjective test to be performed or ending the sequence of subjective tests.

The invention also relates to a method for testing the eyes of an individual, implemented by a computer, comprising a sequence of subjective tests with at least a first and a second predetermined subjective test, leading to the determination of values of one or more refractive features of one or more of the eyes of the individual by performing steps a), b) and c) mentioned above.

Said one or more processors may moreover be programmed to deduce from the refractive features of the eyes of the individual optical features of an ophthalmic lens for improving the visual performance of the individual.

The optical feature can comprise a dioptric optical feature, i.e. an optical feature measured in diopters, or an orientation, i.e. an optical feature representing an angle, for example measured in degrees.

The refractive features of one or more of the eye and, optionally, the optical features of the ophthalmic lens, are determined with the purpose of manufacturing said ophthalmic lens. The ophthalmic lens may then be used to manufacture an optical equipment adapted to the individual for improving its visual performance.

Such optometry device may comprise:
- a display unit generally in the form of a screen to display subjective tests such as an eye chart to be observed by the individual, and
- a phoropter (also called "refractometer") designed for providing one test lens or multiple test lenses with different vision correction powers close to the eye of the individual, through which the individual observes said eye chart, the vision correction powers of the phoropter being changed according to the answers provided by the individual relative to its ability to see optotypes, for example characters, of the eye chart through the test lens providing a vision correction power.

Such phoropter may be of a classical kind with a set of lenses of different powers that can be successively disposed along the optical paths reaching the eyes of the subject, to change the vision correction powers provided to each eye, or of an improved kind, having tunable lenses of continuous variable power disposed along the optical paths reaching the eyes of the subject to change the vision correction powers provided to each eye by the change of the power of the tunable lens. The change of lenses in the case of a standard phoropter or the change of the powers of the tunable lenses in the case of an improved phoropter, and the subjective tests displayed by the screen can be controlled automatically by said computer.

Generally, the screen is placed at 5-6 meters from the phoropter to simulate an observation distance at the infinity, and the eye chart is displayed in a conventional examination room with controlled lighting conditions. The characters of the eye chart and the background of the characters have their own luminance. In this case, the individual observes the image displayed by the screen through the vision correction powers at an observation distance equal to the distance between the screen and the phoropter of 5-6 meters, which could be approximated to the infinity.

Such kind of phoropter and display unit used in the optometry device according to the invention may be those of the device described in document EP3128894 or US20040032568.

For smaller examination rooms, when the screen cannot be placed at 5-6 meters to approximate infinity, or for "compact optometry system" specifically designed to reproduce, in a compact version, an examination room, the screen is placed closer to the phoropter and an optical system allows defining a larger distance of observation than the physical distance separating the screen from the phoropter.

This is the case for the described embodiment, wherein the phoropter belongs to an optometry device having a casing or housing simulating an examination room. The casing or housing encloses a display unit suitable for displaying a test picture to be seen through the phoropter 100, placed at a short distance from the phoropter, but observed by the individual at several observation distances, thanks to an internal optical system.

These components of the optometry device according to the invention may be those of the device described in document WO2020/188104, as described below.

Generally, the optometry device according to the invention comprises for example a refraction test unit having an optical system for providing different vision correction powers close to the eye of the individual, and a display unit adapted to produce a visual test image for the individual's eye. They are controlled by said computer.

The visual test image is visible through an exit aperture of said test unit of the optometry device.

The refraction test unit is interposed between the display unit and the individual's eye. It is movable so that its position may be adjusted in front of the eyes of the individual.

Said refraction test unit may be of any kind known to the man skilled in the art. Such refraction test unit is usually called "phoropter". It is adapted to provide a variable optical correction for the individual's eye looking therethrough.

In particular, it may comprise a classical optical system for presenting different lenses with different optical powers in front of one or each eye of the individual, or no lens or a blank lens with no optical power.

The lenses with different powers are interchanged through a manual or preferably through a motorized command (not represented). These different powers are vision correction powers for the eye of the individual placed nearby.

The refraction test unit preferably comprises an optical system with one or a plurality of lenses with adjustable power, such as liquid lenses.

The refraction test unit is for instance a visual compensation system as described in document WO 2015/155458.

The refraction test unit for example includes a lens having a variable spherical power.

Said variable spherical power lens has for instance a deformable surface. The shape of this surface (in particular the radius of curvature of this surface, and hence the spherical power provided by the lens) can be controlled by moving a mechanical part (such as a ring), which mechanical part may be driven by a motor of the refraction test unit.

The refraction test unit may also include a pair of independently rotatable lenses each having a cylindrical power.

They may each be rotated by action of other motors of the refraction test unit.

The motors are controlled by a control unit such that the combination of the variable spherical power lens and the two cylindrical power lenses provides a desired spherical correction and a desired cylindrical correction to the individual's eye, as explained in document WO 2015/107303.

The various elements of the refraction module (such as the variable spherical power lens, the cylindrical lenses, the motors and the control unit) are enclosed in a housing.

The optometry device preferably includes two visual compensation systems as mentioned above, each such system being situated in front of one of the individual's eyes. The adjustable powers of these visual compensation systems are vision correction powers for the eye of the individual placed nearby.

The refraction test unit optionally comprises one or more element designed to receive the head of the individual and hold it in a predetermined position relative to the refraction test unit 10. This element may for example receive the forehead of the individual. Alternatively or in addition, the refraction test unit could comprise an element to receive the chin of the individual.

Such refraction test unit is well-known and will not be described in more details here.

The display unit comprises:
- a screen adapted to display a test picture used in producing said visual test image and
- at least one optical element having an optical power.

The optometry device is arranged so that a visual test image used in subjective tests of the eyes of the individual may be produced at a variable distance from said exit aperture.

For example, said optical element may be movable between an active position in which it is placed on an optical path of the light emitted by said screen and exiting the device through said exit aperture, and a retracted position in which it remains out of said optical path.

The optical path is the path that a light beam emitted by the screen at a center of the image displayed by the screen takes in traversing the display unit to reach the exit aperture of the refraction test unit.

The distance between the visual test image and the exit aperture of the refraction test unit is then the distance between the exit aperture and the optical position of the visual test image. Said optical element may comprise for example an optical lens.

The distance between said visual test image and said exit aperture is varied at least between a distance of far vision and a different distance of near or intermediate vision. A distance of far vision is typically comprised between infinity and 65-70 centimeters. A distance of intermediate vision is typically comprised between 65-70 and 40 centimeters. A distance of near vision is typically comprised between 40 and 33 centimeters.

Preferably, the relative positions of the screen, the optical element and the exit aperture are adapted to be varied in order for said distance between the visual test image produced and the exit aperture to be continuously varied inside one or several ranges of optical distances between infinity and a near vision distance.

Preferably, said distance between the visual test image produced and the exit aperture may take any value comprised between infinity and a near vision distance.

The optometry device may include a casing adapted to be placed on a table, for instance, or to be mounted on a stand to be placed on a table or on the floor.

The display unit of the optometry device may comprise any kind of displaying device, such as a screen. It may comprise an active digital screen such as a liquid crystal display, or a passive screen and a projection device.

Said one or more processors of the computer are programmed to command said refraction test unit and display unit to perform said sequence of subjective tests and steps a), b) and c).

Said sequence of subjective test is typically determined to provide the refraction features of the eyes of the individual needed to determine the vision correction powers of ophthalmic lenses designed to improve the visual performance of the eyes of the individual.

Said sequence of subjective test comprises at least two subjective tests that are predetermined. These two subjective tests would typically be performed one after the other by known optometry systems of the state of the art. They may belong to the following group of subjective test or comprise any other known subjective test:
- Determining the acuity of one of the eye or of both eyes,
- Fogging and defogging one of the eye to relax accommodation,
- Determining the spherical refraction of one of the eye,
- Determining the cylindrical refraction and or axis of one of the eye,
- Performing a binocular or bi-ocular balance of the eyes,
- Determining the prism or the addition or any dioptric feature of one of the eye.

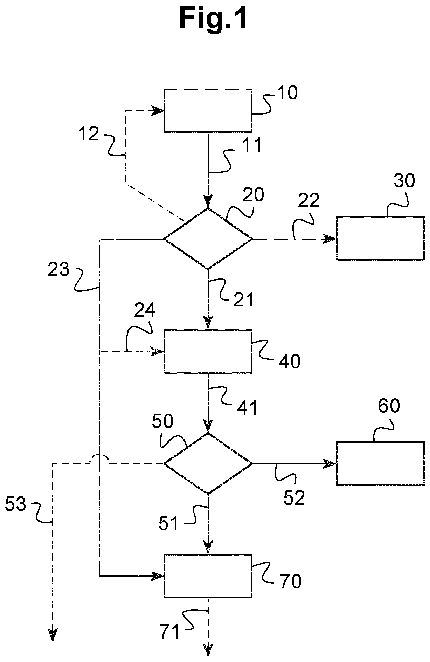

Figure 1 represents schematically a first example of the sequence of a first subjective test 10 and a second subjective test 40 performed by the optometry device according to the invention.

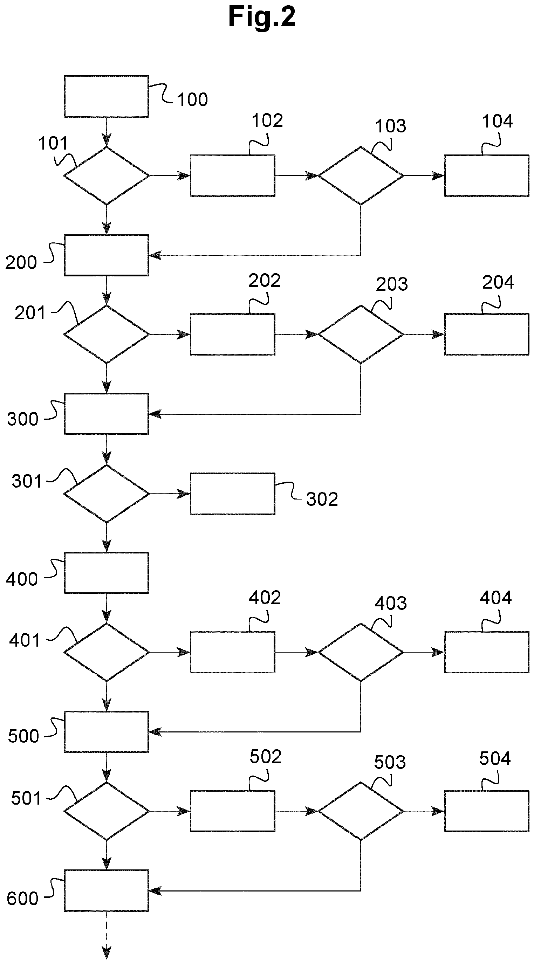

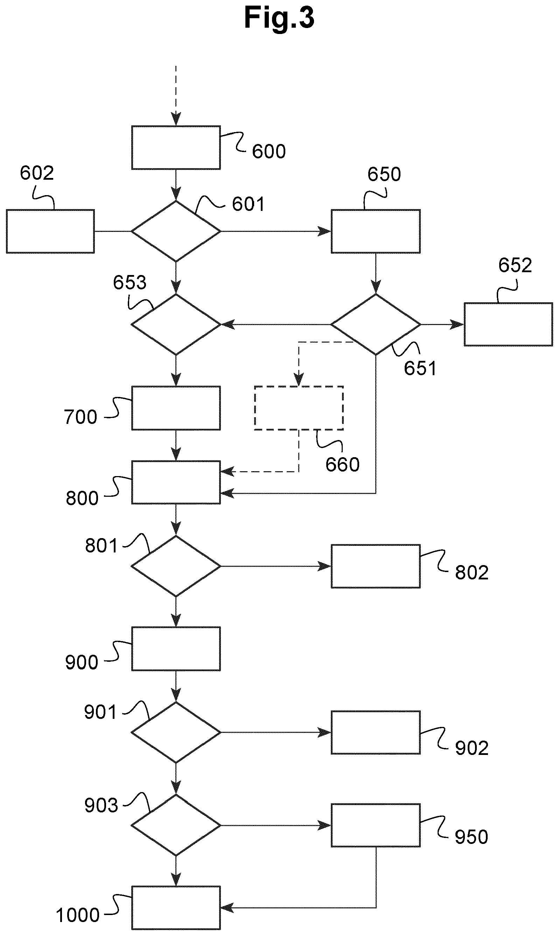

A second example of the sequence of subjective tests comprises for example the following subjective tests, as shown on figure 2 and 3:
- A step of fogging and defogging the right eye (block 100 of figure 2),
- A step of determining a spherical refraction of the right eye (block 200 of figure 2),
- A step of determining a cylindrical refraction and axis of the right eye (block 300 of figure 2),
- A step of fogging and defogging the left eye (block 400 of figure 2),
- A step of determining a spherical refraction of the left eye (block 500 of figure 2),
- A step of determining a cylindrical refraction and axis of the left eye (block 600 of figure 2 or 3),
- A step of adjusting a balance of the eyes of the individual (block 700 of figure 3),
- A step of adjusting the spherical refraction of the right and left eyes in binocular vision conditions (block 900 of figure 3).

Said one or more refraction features of the eyes of the individual may comprise a spherical refraction of the left and/or right eye, a cylindrical refraction and/or axis of the left and/or right eye, an addition of the left and/or right eye, a prism value of the left and/or right eye .

In the example of the sequence of subjective tests mentioned above, performing the sequence leads to determining values of spherical refraction, cylindrical refraction and axis of both eyes of the individual.

Here, the optical feature of the ophthalmic lens deduced may comprise for example one or more of the following: a sphere power, a cylinder power, a cylinder axis, a difference in sphere between right and left eyes.

Alternatively, said sequence of subjective test may comprise other subjective tests as known from the skilled person.

The subjective tests of the sequence are stored in one or more memories of the computer. The one or more processors are programmed to take them into account successively, in a specific order.

Each subjective test of the sequence is predetermined in the sense that the type of test is determined as well as the parameters of this test.

As mentioned in introduction, each subjective test comprises several steps, hereafter called trials, during each of which the individual is required to compare two different optical situations taking into account a test value of an optical feature of a test lens placed in front of his eye. Depending on the feedback of the individual on this comparison, otherwise designated as the answer of the individual, the eye-care professional increments the test value and, in the next trial of the subjective test, present the individual with two new different optical situations based on the incremented test value. This process is repeated until a specific answer or combination of answers is given by the individual. The value of the refractive feature of the eye is then usually determined as a function of the test value used in the last trial of said subjective test.

In particular, said first and second subjective test each comprise a plurality of trials wherein a visual performance of the individual placed in two different optical situations is assessed.

Each subjective test may be associated with the refractive feature sought to be determined and with a set of parameters indicating how the subjective test is performed. The parameters may include the value of the increment between two successive test value, the initial test value used at the beginning of the subjective test, the type of optical situations compared, the features of these optical situations...

Some subjective tests may be performed with different optical situations that are provided to the individual. These optical situations may have different characteristics depending on the optical feature determined through the corresponding subjective test. The optometry device may consequently comprise different optical elements.

The subjective tests for determining the spherical refraction of one or more of the eye may for example be carried out by the following different methods where different types of optical situations are used:
- A duochrome test comprising placing, in front of the eye of the individual, a trial lens having an optical feature equal to a test value and displaying, here simultaneously, targets comprising a sign placed on a red or green environment,
- An acuity test with or without fogging and defogging of the eye, i.e. with or without accommodation relaxation, comprising
   ∘ placing successively, in front of the eye of the individual, two trial lenses having an optical feature equal to two different test values (different spheres) and displaying a target, or
   ∘ placing, in front of the eye of the individual, a trial lens having an optical feature equal to a test value and displaying two or more targets of different size.

The subjective test for determining the cylindrical refraction and/or axis may comprise a cross cylinder test comprising displaying a target having multiple elements and adding, in front of the eye of the individual, a cross cylinder placed successively in two different positions depending on a cylinder axis test value.

The subjective test for adjusting the spherical refraction of the eyes to achieve binocular balance or bi-ocular balance may comprise :
- placing simultaneously different trial lenses in front of the right and left eye of the individual and displaying a different target for each eye,
- placing simultaneously different trial lenses in front of the right and left eye of the individual and displaying a single target for both eyes, seen independently by each eye thanks to a polarizer and beam splitter or a prism.

The corresponding optical device may therefore comprise optical means for achieving each of these subjective tests. These means are well known from the skilled person and will not be described in detail in the present description.

Any two successive tests of the different subjective tests described above may be considered as the first and the second predetermined subjective tests previously defined.

Said one or more processors of the optometry device according to the invention are programmed to first perform said first predetermined subjective test (block 10 of figure 1). By performing the first predetermined subjective test, a provisory value of a first refraction feature of the eye of the individual is determined.

The provisory value of the first refraction feature may be for example:
- a provisory value of the spherical refraction of the eye after fogging/defogging,
- a provisory value of the spherical refraction of the eye without fogging/defogging,
- a provisory value of the cylindrical refraction or axis of the eye,
- a provisory value of the difference in spherical refraction between the two eye,
- a provisory value of the addition or prism of the eye.

After determining said provisory value, preferably immediately after, the one or more processors are programmed to perform a verification test for detecting a potential error in said provisory value of the first refraction feature (arrow 11 and block 20 of figure 1).

Said verification test may for example comprise at least one of the following:
b1) comparing the provisory value of the first refraction feature determined through said first subjective test to a refraction threshold value, and
b2) comparing an acuity value of at least one of the eyes of the individual to an acuity threshold value.

When step b1) is performed during step b), said one or more processors are programmed to determine said refraction threshold value based on a previous value of the first refraction feature determined before performing said first subjective test.

For example, said verification test may comprise comparing an initial value of the refraction feature used to start the subjective test to the provisory value determined at the end the subjective test. Said initial value of the refraction feature may be determined through an objective measurement or be deduced from the current optical equipment of the individual.

For example, said first subjective test may be a step of fogging and defogging one of the eye of the individual. The initial value of the spherical refraction of the eye may be determined with an autorefractometer and compared to the provisory value of the spherical refraction of the eye after fogging/defogging.

If the difference between the initial value of the spherical refraction and the provisory value of the spherical refraction of the eye after fogging/defogging is lower than a predetermined allowed difference, the first subjective test is validated as correct. Otherwise, the one or more processors are programmed to detect a possible error. The predetermined allowed difference is for example equal to 2 diopters (D).

In this example, the threshold value is the sum of the initial value and the predetermined allowed difference.

When step b2) is performed during step b), said one or more processors are programmed to either:
- use a value of the acuity of the eye determined during the first subjective test or
- perform an additional visual acuity test at the end of the first subjective test to determine said acuity value.

Said acuity value may comprise a monocular acuity value of said eye or a binocular acuity value.

Said acuity threshold value comprises one of the following:
- an initial value of visual acuity of at least one of the eyes of the individual determined before said first subjective test,
- a reference value of visual acuity of the other eye of the individual determined before or after said first subjective test,
- a target value of visual acuity.

Said acuity threshold value may in particular comprise a predetermined target value of visual acuity, for example a value of 10/10^{th}.

Said acuity threshold value may comprise a monocular acuity value or a binocular acuity value.

Step b2) may comprise comparing said acuity value of the tested eye to the predetermined acuity value, to the acuity value of the other eye or to a binocular acuity value.

In step b), said one or more processors are programmed to detect a potential error in said provisory value:
- when the first refraction feature is above said refraction threshold value or
- when the acuity value is less than said acuity threshold value or
- when the first refraction feature is above said refraction threshold value and the acuity value is less than said acuity threshold value.

In step c), said one or more processors are programmed to determine a next subjective test to be performed or ending the sequence of subjective tests, based on the result of said verification test.

More precisely, when no potential error is detected in step b), said one or more processors are programmed to record said provisory value as a confirmed value of said first refractive feature and perform said predetermined second subjective test.

This case is represented by arrow 21 and block 40 of figure 1.

When a potential error is detected in step b), said one or more processors are programmed to determine, in step c), the next subjective test to be performed by:
- cancelling the first subjective test and performing either one of said second predetermined subjective test without any modification of the second predetermined subjective test (arrow 24 of figure 1) and a modified second subjective test testing the same second refraction feature of the same eye with a different method,
   or
- repeating said first subjective test without modification of the first subjective test (arrow 12 of figure 1) or performing a modified first subjective test testing the same first refraction feature of the same eye with a different method.

The modified first or second subjective test is associated with the same first or second refractive feature sought to be determined and with a modified set of parameters, comprising at least one parameter different from the corresponding parameter of set of parameters of the first or second subjective tests.

In the following, when the optometry device of the invention is adapted to place said individual in different types of optical situations to perform said first or second subjective test, said predetermined first or second subjective test is associated with a predetermined type of optical situations. Said modified first or second subjective test is for example associated with a different type of optical situations.

The one or more processors may be programmed to perform a new verification step after performing the modified first subjective test. When a potential error is detected, the one or more processors may be programmed to performed yet another modified first subjective test, a modified second subjective test or to end the sequence.

Alternatively, the one or more processors are programmed to end the sequence of subjective tests, as represented by arrow 22 and block 30 of figure 1.

In a case where said sequence of subjective tests comprises a third predetermined subjective test (block 70 of figure 1), when a potential error is detected in step b), said one or more processors may be programmed to determine, in step c), the next subjective test may comprise performing said third predetermined subjective test without performing said second predetermined subjective test (arrow 23 of figure 1).

The next subjective step performed after the first subjective test is therefore not necessarily the second predetermined subjective test, as it would be in a standard sequence of the state of the art.

In the example of figure 1, at block 20, the first refraction feature is above said refraction threshold value and the acuity value is less than said acuity threshold value so that a potential error is detected in the step b).

Any couple of two predetermined subjective tests to be successively performed during said sequence may play the role of the first and second predetermined subjective tests.

In the example of figure 1, step a), b) and c) may be applied to the second and third predetermined subjective tests: by performing the second predetermined subjective test 40, a provisory value of the second refraction feature is determined, and the one or more processors are programmed to perform a verification step (arrow 41, block 50) for detecting a potential error in said provisory value of the second refraction feature.

When no potential error is detected, the one or more processors are programmed to perform the third predetermined subjective test (arrow 51, block 70. Otherwise, in the example of the figure 1, when a potential error is detected, the one or more processors are programmed to end the sequence (arrow 52, block 60), or perform a modified third subjective test (arrow 53).

In a first embodiment of the invention detailed below, the sequence comprises a first subjective test comprising the fogging and defogging of right eye, a second subjective test comprising the determination of the spherical refraction of the right eye and a third subjective test comprising the determination of the cylindrical refraction and axis of the eye.

After performing the first subjective test and before performing the second subjective test, the one or more processors are programmed to perform a verification step by comparing the provisory value of spherical refraction of the right eye determined at the end of the defogging process with an initial value of the spherical refraction determined at the beginning of the test, before fogging the right eye. If the difference is for example over 2 D, a potential error is detected. Alternatively, the one or more processors may be programmed to determine the final value of the visual acuity of the right eye at the end of the first subjective test and compare it to a target value of 10/10^{th}. If the final value of the visual acuity is less than 10/10th, a potential error is detected. The first subjective test is canceled, and the second subjective test is performed or a modified first subjective test of fogging/defogging is performed, or the sequence is ended.

Said first subjective test uses a standard fogging/defogging method. Each trial of this first subjective test typically comprises displaying two targets of different size and providing the individual with a trial lens having a trial sphere power value.

This subjective test is a monocular test.

In a first optical situation, the individual looks at a first target through the trial lens. The size of the first target, seen at the viewing distance of the individual, corresponds to a first acuity value.

In a second optical situation, the individual looks at a second target, smaller than the first target, through the same trial lens. The size of the second target, seen at the viewing distance of the individual, corresponds to a second acuity value.

During each trial, the individual is asked to look at the two targets through the trial lens having a trial sphere power value equal to the test value, and to identify with which target the sign appears sharper.

The two targets are for example lines of letters of an acuity chart. For example, for the first trial, the first target is a line at 4/10 and the second target a line at 8/10. The size of the first and second target may change from a trial to the next.

The individual is asked to assess which one of the two first and second optical situations provides higher visual performance according to his perception. To obtain this information, depending on the subjective test performed, the eye-care professional may for example ask, *"in which situation do you better* see *the target"* or *"which target do you better see"* or-"what is the smallest target you can read?".

The answer of the individual is inputted in the computer.

The initial test value can for example be determined as the current correction of the individual or based on an objective or subjective measurement, plus a determined dioptric value, for example superior to 1 D. This addition of a determined dioptric value corresponds to an initial fogging step for relaxing the individual accommodation.

Then, as long as the individual can clearly read the second target, the test value is increased.

This increase of the test value corresponds to the fogging step.

Then, at the moment when the individual can clearly read only the first target, i.e. when the result of the trial is the third answer, the test value is decreased.

This first decrease of the test value is the beginning of a second part of the test: the defogging step.

Then, as long as the individual can clearly read only the first target, i.e. when the result of the successive trials is the third answer 300, the test value is decreased.

Here, during the defogging step, the variation increment is preferably decreased from one trial to the next.

The modified first subjective test of fogging/defogging may comprise an alternative method for defogging the eye, where the time needed for the individual to compare the optical situations is taken into account or the individual reading speed when he reads the optotypes displayed. The level of certainty of the individual may be taken into account, based for example on the method described in document EP3272274

If no potential error is detected, the provisory value is validated and put in memory. The second subjective test is performed.

In a second embodiment of the invention that will be detailed below, the sequence comprises a first subjective test comprising the determination of the spherical refraction of the right eye, a second subjective test comprising the determination of the cylindrical refraction and axis of the right eye and a third subjective test comprising the determination of the visual acuity of the right eye.

After performing the first subjective test and before performing the second subjective test, the one or more processors are programmed to perform a verification step by comparing the provisory value of spherical refraction of the right eye determined at the end of the first subjective test with an initial value of the spherical refraction determined at the beginning of the test or before the test. If the difference is over 1 D, a potential error is detected. A second verification step is performed: the one or more processors are programmed to determine the final value of the visual acuity of the right eye at the end of the first subjective test and compare it to its initial value at the beginning of the first subjective test. If the final value of the visual acuity is less than its initial value, a potential error is detected. A modified first subjective test is performed before performing the second subjective test or the sequence is ended.

Said first subjective test uses an acuity test using optotypes for spherical refraction determination method. Each trial of this first subjective test typically comprises displaying one target and asking the individual to compare a first trial lens with a first trail sphere power value and a second trial lens with a second trial sphere power values.

This subjective test is a monocular test.

In the first optical situation, the individual looks at said target through the first trial lens.

In the second optical situation, the individual looks at the same target, through the second trial lens.

During each trial, the individual is asked to look at the target through the first and second trial lenses , and to identify with which one of said first and second trial lens the sign appears sharper.

The individual is for example asked to assess which one of the two first and second optical situations corresponding to the use of the first and second trial lenses provides higher visual performance according to his perception. To obtain this information, depending on the subjective test performed, the eye-care professional may for example ask, *"in which situation do you better* see *the target"* or *"which target do you better see"*.

The subsequent trial is determined by the one or more processors based on the answer of the individual in the previous trials.

The modified first subjective test may comprise a duochrome test in which the optical situations correspond to displaying signs on a red or a green background.

In such a duochrome test, each trial comprises displaying a target comprising a sign placed in a red and green environment and providing the individual with a trial lens having a trial sphere power value.

This subjective test is a monocular test.

In the first optical situation, a first target comprises a sign displayed in a uniform green background. Said sign comprises for example an optotype or a geometrical shape.

In the second optical situation, a second target comprises a sign displayed in a uniform red background. Said sign comprises for example an optotype or a geometrical shape. The second sign may be identical to the first sign.

During each trial, the individual is asked to look at the two targets through the trial lens having a trial sphere power value equal to the test value, and to identify with which target the sign appears sharper.

An initial test value can for example be determined as the current correction of the individual or based on an objective or subjective measurement.

If the individual sees the sign sharper on the green background than on the red background, it means that trial sphere power value of the trial lens is not sufficient to provide an appropriate correction for the eye of the individual tested. The one or more processors are programmed to increased it in the next trial.

If the individual sees the sign sharper on the red background than on the green background, it means that sphere power of the trial lens is too strong to provide an appropriate correction for the eye of the individual tested. The one or more processors are programmed to decrease it in the next trial.

If the individual sees the sign as equally sharp on the green and on the red background, the sphere power of the trial lens is appropriate.

When no potential error is detected, the provisory value is validated and put in memory. The second subjective test is performed.

This second subjective test is for example a cross cylinder test.

During each trial, the individual is asked to look at a target through a trial lens which is known as "Jackson Cross Cylinder" and referred to here as the cross cylinder.

In the first optical situation, the cross cylinder is positioned in a first orientation. In the second optical situation, the cross cylinder is positioned in a second orientation.

For the individual, in both optical situations, the target seen through the cross cylinder appears unclear. During the subjective test, the eye-care professional may ask *"in which situation is the target less blurred?".* The one or more processors are programmed to increment the orientation angle to improve the clarity of the target.

After performing the second subjective test and before performing the third subjective test, the one or more processors are programmed to perform a verification step by comparing the provisory value of cylindrical refraction of the right eye determined at the end of the second subjective test with an initial value of the cylindrical refraction determined at the beginning of the test or before the test. If the difference is over 1 D, a potential error is detected. A second verification step is performed: the one or more processors are programmed to determine the final value of the visual acuity of the right eye at the end of the second subjective test and compare it to its initial value at the beginning of the second subjective test. If the final value of the visual acuity is less than it initial value, a potential error is detected. A modified second subjective test is performed or the sequence is ended.

The modified second subjective test may for example comprise a cross cylinder test with a modified trial power or axis, a different sequence of trial power and/or axis, a different target or different optical situations.

It is also possible to modify the target to display a target depending on the initial acuity of the individual. The question asked may then be "in which position do you best see the target?".

In a third embodiment of the invention as detailed below, the sequence comprises a first subjective test comprising the adjustment of a balance between the eyes performed while providing two independent test images to the eyes of the individual, a second subjective test comprising the determination of the binocular acuity of the eyes and a third subjective test comprises performing a binocular spherical refraction determination.

The first subjective test is for example a standard bi-ocular balance test.

In the first optical situation, the individual looks with his left eye at a target, through a left trial lens. The left trial lens is characterized by a left trial sphere power value.

In the second optical situation, the individual looks with his right eye at the same target through a right trial lens. The right trial lens is characterized by a right trial sphere power value which differs from the left trial sphere power value.

During each trial, the individual is asked to look successively at the target with his left eye through the left trial lens and then with his right eye through the right trial lens.

The initial test value can for example be determined as the difference in sphere power between right and left eye based on the current correction of the individual or based on two objective monocular measurements, one for the right eye and one for the second eye.

If the individual sees the target sharper with the left trial lens, i.e. with the left eye, than with the right trial lens, the lens power provided to the left eye is increased and the lens power provided to the right eye is decreased for the second trial.

If the individual sees the target sharper with the right trial lens, i.e. with the right eye, than with the left trial lens, the lens power provided to the right eye is increased and the lens power provided to the left eye is decreased for the second trial.

Preferentially, the left trial sphere power value and the right trial sphere power value are modified in a symmetrical way by a same amount.

After performing the first subjective test and before performing the second subjective test, the one or more processors are programmed to perform a verification step by comparing the provisory value of spherical refraction of the right and left eyes determined at the end of the balance adjustment with an initial value of the spherical refraction of each eye determined at the beginning of the test. If the difference between the provisory value and the corresponding initial value for at least one of the eye is over 1 D, a potential error is detected. The first subjective test is canceled, and a modified first subjective test of balance adjustment is performed, or the second predetermined subjective test is directly performed.

The modified first subjective test may use red/green target and/or masks adapted to be placed in front of one eye to allow the individual to look at the target only with the other eye.

If no potential error is detected, the provisory value is validated and put in memory. The second predetermined subjective test of binocular acuity is performed.

After performing the second subjective test and before performing the third subjective test, the one or more processors are programmed to perform a verification step by comparing the provisory value of binocular acuity determined with values of the monocular acuity of each eye determined at the beginning of the test. If the difference between the provisory value and the corresponding initial value for at least one of the eye is over 1 D, a potential error is detected. A modified second subjective test of binocular acuity is performed, or the sequence is ended.

If no potential error is detected, the provisory value of binocular acuity is validated and put in memory. The third predetermined subjective test is performed.

Besides, said one or more processors may be programmed to perform an additional preliminary step before at least one of the subjective tests of said sequence of subjective tests, checking that predetermined conditions on the individual are met and determining whether said at least one subjective test is cancelled or modified based on the result of this additional preliminary step.

An example of implementation of the additional preliminary step of the invention is the following. A sequence comprises a first subjective test comprising the determination of the visual acuity of the right eye, a second subjective test comprising the determination of the visual acuity of the left eye and a third subjective test comprising performing a step of bi-ocular balance by testing each eye while the other remains open.

After performing the first and second subjective test and before performing the third subjective test, the one or more processors are programmed to perform an additional preliminary step of comparing the visual acuity of the left and right eyes. If they are different, the third subjective test is skipped or a modified third subjective test is performed, for example using a duochrome test instead of visual acuity tests using optotypes.

In another example of implementation of said additional preliminary step, said one or more processors are programmed to compare the age of the individual with a threshold value of age.

The invention also relates to a program product comprising instructions which, when the program is executed by a computer, cause the computer to carry out the steps of the method described above.

In the following, a global example of the determination of the refraction features of the eyes of an individual will be described to show how the optometry device of the invention and the method of the invention may be used.

The sequence comprises the subjective tests listed in the second example of sequence described above, in reference to figures 2 and 3. Except when otherwise stated, the refraction features of the eyes are determined in far vision conditions.

In a preliminary step not represented on figure 2, the initial monocular acuity of the right and left eyes and, optionally, the initial binocular acuity of the eyes are determined. During this preliminary step, the individual wears its current optical equipment or no optical equipment. These initial values of acuity could be those taken into account for comparisons in the verification steps described hereafter.

In the first subjective test of block 100, a step of fogging and defogging the right eye is performed. The fogging/defogging steps described above may be used.

Then, the one or more processors are programmed to perform a first verification step (block 101) by comparing the provisory value of spherical refraction of the right eye determined at the end of the defogging process with an initial value of the spherical refraction determined before or at the beginning of the first subjective test, before fogging the right eye. If the difference is over 2 D, the one or more processors are programmed to perform a second verification test comprising a a visual acuity test (block 102 of figure 2) by determining the monocular acuity of the right eye and comparing it (block 103 of figure 2) to the initial value of acuity of the right eye or to a target value of 10/10^{th}.

When the monocular acuity of the right eye is less than its initial value or less than the target value, a potential error is detected. The sequence is then ended (block 104 of figure 2).

When the difference between the provisory value of spherical refraction of the right eye and the value of the spherical refraction determined before or at the beginning of the first subjective test is less than 2 D or when the monocular acuity of the right eye at the end of the first subjective test is superior or equal to the corresponding initial value or target value, the provisory value of spherical refraction of the right eye determined at the end of the defogging process is validated and put in memory. The one or more processors are then programmed to perform the second subjective test.

The second subjective test comprises a step of determining a spherical refraction of the right eye (block 200 of figure 2). During this subjective test, the spherical refraction is more finely adjusted. Another provisory value of spherical refraction of the right eye is determined.

Then, the one or more processors are programmed to perform a third verification step (block 201) by comparing the provisory value of spherical refraction of the right eye determined at the end of the second subjective test with the value of the spherical refraction determined at the beginning of the second subjective test. If the difference is over 2D, the one or more processors are then programmed to perform a fourth verification step comprising a visual acuity test (block 202 of figure 2). They are programmed to determine the monocular acuity of the right eye and compare it (block 203 of figure 2) to the initial value of acuity of the right eye or to a target value of 10/10^{th}.

When the monocular acuity of the right eye is less than its initial value or less than the target value, a potential error is detected. The sequence is then ended (block 204 of figure 2).

When the difference between the provisory value of spherical refraction of the right eye and the value of the spherical refraction determined before or at the beginning of the second subjective test is less than 2 D or when the monocular acuity of the right eye at the end of the second subjective test is superior or equal to the corresponding initial value or target value, the provisory value of spherical refraction of the right eye determined at the end of the second subjective test is validated and put in memory. The one or more processors are then programmed to perform the third subjective test.

The third subjective test comprises a step of determining a cylindrical refraction and axis of the right eye (block 300 of figure 2). This third subjective test corresponds to a cross cylinder test. The cross cylinder test described above may be used.

Then, the one or more processors are programmed to perform a fifth verification step (block 301) by comparing the provisory value of cylindrical refraction or axis of the right eye determined at the end of the third subjective test with the value of the cylindrical refraction or axis determined before or at the beginning of the third subjective test. When the difference is for example over 1.25D, preferably over 1.75D, a potential error is detected.

The sequence is then ended (block 302 of figure 2).

When the difference is for example less than 1.25D or less than 1.75D, the provisory value of cylindrical refraction of the right eye determined at the end of the third subjective test is validated and put in memory.

The one or more processors are then programmed to repeat for the left eye the steps described above for the right eye, in the fourth, fifth and sixth subjective tests (blocks 400, 500 and 600 of figure 2).

In the fourth subjective test of block 400, a step of fogging and defogging the left eye is performed.

Then, the one or more processors are programmed to perform a sixth verification step (block 401) by comparing the provisory value of spherical refraction of the left eye determined at the end of the defogging process with an initial value of the spherical refraction of the left eye determined at the beginning of the test, before fogging the left eye.

When the difference is over 2 D, the one or more processors are programmed to perform a seventh verification step comprising a visual acuity test (block 402 of figure 2). They are programmed to determine the monocular acuity of the left eye and compare it (block 403 of figure 2) to the initial value of acuity of the left eye or to a target value of 10/10^{th}. When the monocular acuity of the left eye is less than its initial value or less than the target value, a potential error is detected. The sequence is then ended (block 404 of figure 2).

When the difference between the provisory value of spherical refraction of the left eye and the value of the spherical refraction determined before or at the beginning of the fourth subjective test is less than 2 D or when the value of the monocular acuity of the left eye at the end of the fourth subjective test is superior or equal to the corresponding initial value or target value, the provisory value of spherical refraction of the left eye determined at the end of the defogging process is validated and put in memory. The one or more processors are then programmed to perform the fifth subjective test.

The fifth subjective test comprises a step of determining a spherical refraction of the left eye (block 500 of figure 2). During this subjective test, the spherical refraction is more finely adjusted. Another provisory value of spherical refraction of the left eye is determined.

Then, the one or more processors are programmed to perform an eight verification step (block 501) by comparing the provisory value of spherical refraction of the left eye determined at the end of the second subjective test with the value of the spherical refraction of the left eye determined before or at the beginning of the fifth subjective test. When the difference is over 2 D, the one or more processors are programmed to perform an ninth verification step comprising a visual acuity test (block 502 of figure 2). They are programmed to determine the monocular acuity of the left eye and compare it (block 503 of figure 2) to the initial value of acuity of the left eye or to a target value of 10/10^{th}.

When the monocular acuity of the left eye is less than its initial value or less than the target value, a potential error is detected. The sequence is then ended (block 504 of figure 2).

When the difference is less than 2 D or when the value of the monocular acuity of the left eye at the end of the fifth subjective test is superior or equal to the corresponding initial value or target value, the provisory value of spherical refraction of the left eye determined at the end of the fifth subjective test is validated and put in memory. The one or more processors are programmed to perform the sixth subjective test.

The sixth subjective test comprises a step of determining a cylindrical refraction and axis of the left eye (block 600 of figure 2). This sixth subjective test corresponds to a cross cylinder test.

Then, the one or more processors are programmed to perform a tenth verification step (block 601 of figure 3) by comparing the provisory value of cylindrical refraction or axis of the left eye determined at the end of the sixth subjective test with the value of the cylindrical refraction determined at the beginning of the sixth subjective test. If the difference is over 1.50D, a potential error is detected.

The sequence is then ended (block 602 of figure 3).

When no potential error has been detected for any of the preceding subjective tests of the sequence, the provisory value of cylindrical refraction of the left eye determined at the end of the sixth subjective test is validated and put in memory.

The one or more processors are then programmed to perform an additional preliminary step (block 653 of figure 3) for checking that predetermined conditions on the individual are met to perform the seventh subjective test comprising an ocular balance test of the eyes.

The additional preliminary step comprises a double vision test design to check that the individual is able to see simultaneously two independent images, each image being seen with one of the eyes.

When the additional preliminary step indicates that the individual is able to have a double vision, the one or more processors are programmed to perform the seventh subjective test (block 700 of figure 3).

This seventh subjective test is an ocular balance test using a single target and a prism (or polarizers) to display two images of the single target, one of the two images being provided to each of the eyes. Each eye sees a different image of the target, independently from the other eye and simultaneously with the image of the target seen by the other eye. The individual therefore has a double vision of the target. The individual is asked to compare the two images of the target seen simultaneously to indicate which of them is seen more clearly. Each eye is provided with a trial lens having a different trial power.

The answers of the individual are inputted in the computer and taken into account by the one or more processors which are programmed to increment the trial power of the trial lenses until the two images are seen equally clearly by the individual.

When at least one potential error has been detected for any of the preceding subjective tests of the sequence, or when the visual acuity test performed since the beginning of the sequence have never determined a visual acuity superior or equal to 10/10th, the one or more processors are programmed to perform a visual acuity test (block 650 of figure 3). The values of the monocular visual acuity of the right and left eyes are determined and compared (block 651 of figure 3) between them and to the initial values of monocular visual acuity of the corresponding eye or to target values.

When at least one of the values of monocular visual acuity of the right and left eyes determined are less than the initial value for the corresponding eye or less than the target value, the sequence is ended (block 652 of figure 3).

When both values of monocular visual acuity of the right and left eyes determined are superior or equal to the initial value for the corresponding eye or to the target value and the two values of monocular visual acuity of the right and left eyes determined are equal, the one or more processors are programmed to validate and put in memory the cylindrical refraction and/or axis of the left eye and perform said additional preliminary step of checking the double vision ability of the individual (block 653 of figure 3).

When both values of monocular visual acuity of the right and left eyes determined are superior or equal to the initial value for the corresponding eye or to the target value but the two values of monocular visual acuity of the right and left eyes determined are different from each other, the one or more processors are programmed to skip the seventh subjective step of binocular balance and perform a binocular visual acuity test (block 800 of figure 3).

Alternatively, in the last case, said one or more processors may be programmed to perform a modified seventh subjective test (block 660 of figure 3) by performing the balance for the right and the left eye separately, without using double vision.

The modified seventh subjective test also uses a single target and a prism or polarizers to provide two distinct images of the target, each eye of the individual being simultaneously provided with one image of said two distinct images, independently from the other eye. The target is however modified to comprise a red and a green area, such that each of the eye sees both color.

The individual is then asked to compare the vision of the red and green parts of the target with each eye. The individual therefore is not asked to compare the vision of the two images of the target seen by the two eyes. The test may then be performed even if the individual does not have double vision. The one or more processors are then programmed to increment the trial powers of the trial lenses to achieve similar vision of the red and green targets by the two eyes.

The one or more processors are then programmed to perform a binocular visual acuity test and perform an eleventh verification test (block 801) by comparing the value of the binocular visual acuity with a target value.

When the value determined is less than the target value, the one or more processors are programmed to end the sequence (block 802 of figure 3).

Otherwise, the one or more processors are programmed to perform the eight subjective test of determining the binocular spherical refraction of the eyes (block 900 of figure 3).

The one or more processors are then programmed to perform a twelfth verification test (block 901) comprising determining a final value of the monocular visual acuity of the right and left eye and a final value of the binocular visual acuity of the eyes and comparing the final values with previously determined values, for example with the initial values determined before the first subjective test.

When any one of the final values is less than the corresponding initial value, the eight subjective tests are canceled, and the sequence is ended (block 902).

Otherwise, the one or more processors are programmed to perform another additional preliminary step (block 903) of comparing the age of the individual to an age threshold. If the age of the individual is over said threshold, for example 40 years, the one or more processors are programmed to perform additional subjective tests for determining the refraction features of the eyes in near vision conditions (block 950).

When the age is below said threshold, the sequence is complete.

In a general manner, when the sequence is ended, the individual is referred to an eye care professional for a manual sequence of subjective test. The one or more processors of the optometry device are programmed to output the values of the refraction features of the eyes that have been validated previously before the end of the sequence.

## Claims

1. Optometry device for testing the eyes of an individual, comprising a computer with one or more processors programmed to perform a sequence of subjective tests comprising at least a first (10) and a second (40) predetermined subjective test, leading to the determination of values of one or more refractive features of one or more of the eyes of the individual wherein said one or more processors are programmed to:
a) perform said first predetermined subjective test (10), thereby determining a provisory value of a first refraction feature of the eye of the individual,
b) at the end of said first predetermined subjective test, perform a verification test (20) for detecting a potential error in said provisory value of the first refraction feature determined in step a),
c) based on the result of said verification test, determine a next subjective test (40, 70) to be performed or ending (30) the sequence of subjective tests.

2. Optometry device according to claim 1, wherein, in step b), said one or more processors are programmed to:
- compare the provisory value of the first refraction feature determined through said first subjective test to a refraction threshold value,
and/or
- compare an acuity value of at least one of the eyes of the individual to an acuity threshold value.

3. Optometry device according to claim 2, wherein, in step b), said one or more processors are programmed to detect a potential error in said provisory value:
- when the first refraction feature is above said refraction threshold value or
- when the acuity value is less than said acuity threshold value or
- when the first refraction feature is above said refraction threshold value and the acuity value is less than said acuity threshold value.

4. Optometry device according to any one of claims 2 and 3, wherein said one or more processors are programmed to determine said refraction threshold value based on a previous value of the first refraction feature determined before performing said first subjective test.

5. Optometry device according to any one of claims 2 to 4, wherein said one or more processors are programmed to perform, in step b), an additional visual acuity test at the end of the first subjective test to determine said acuity value.

6. Optometry device according to any one of claims 2 to 5, wherein, when a potential error is detected in step b), said one or more processors are programmed to determine, in step c), the next subjective test to be performed by :
- cancelling the first subjective test and performing either one of said second predetermined subjective test without any modification of the second predetermined subjective test and a modified second subjective test testing the same second refraction feature of the same eye with a different method,
or
- repeating said first subjective test without modification of the first subjective test or performing a modified first subjective test testing the same first refraction feature of the same eye with a different method.

7. Optometry device according to claim 6, wherein, said first or second subjective test comprises a plurality of trials wherein a visual performance of the individual placed in two different optical situations is assessed and said optometry device is adapted to place said individual in different types of optical situations to perform said first or second subjective test, said predetermined first or second subjective test is associated with a predetermined type of optical situations and said modified first or second subjective test is associated with a different type of optical situations.

8. Optometry device according to any one of claims 2 to 5, wherein said sequence of subjective tests comprises a third second predetermined subjective test, and, when a potential error is detected in step b), said one or more processors are programmed to determine, in step c), the next subjective test to be performed by:
- performing said third predetermined subjective test without performing said second predetermined subjective test.

9. Optometry device according to any one of claims 2 to 8, wherein said acuity threshold value comprises one of the following:
- an initial value of visual acuity of at least one of the eyes of the individual determined before said first subjective test,
- a reference value of visual acuity of the other eye of the individual determined before or after said first subjective test,
- a target value of visual acuity.

10. Optometry device as claimed in any one of claims 1 to 9, wherein said one or more processors are programmed to perform the following step:
- when no potential error is detected in step b), recording said provisory value as a confirmed value of said first refractive feature and performing said predetermined second subjective test.

11. Optometry device according to any one of claims 1 to 10, wherein said one or more processors are programmed to perform an additional preliminary step before at least one of the subjective tests of said sequence of subjective tests, checking that predetermined conditions on the individual are met and determining whether said at least one subjective test is cancelled or modified based on the result of this additional preliminary step.

12. Optometry device according to claim 11, wherein, in said additional preliminary step, said one or more processors are programmed to compare a value of the visual acuity of the right eye with a value of the visual acuity of the left eye or compare the age of the individual with a threshold value of age.

13. Method for testing the eyes of an individual, implemented by a computer, comprising a sequence of subjective tests with at least a first (10) and a second (40) predetermined subjective test, leading to the determination of values of one or more refractive features of one or more of the eyes of the individual by:
a) performing said first subjective test (10), thereby determining a provisory value of a first refraction feature of the eye of the individual,
b) at the end of said first subjective test, performing a verification test (20) for detecting a potential error in said provisory value of the first refraction feature determined in step a),
c) based on the result of said verification test, determining a next subjective test (40, 70) to be performed or ending (30) the sequence of subjective tests.

14. Method according to claim 13, wherein said verification test comprises:
- comparing the provisory value of the first refraction feature determined through said first subjective test to a refraction threshold value,
and/or
- comparing an acuity value of at least one of the eyes of the individual to an acuity threshold value.

15. Computer program product comprising instructions which, when the program is executed by a computer, cause the computer to carry out the steps of the method of claim 13 or 14, leading to the determination of values of one or more refractive features of one or more of the eyes of the individual.
